# EUROPEAN PATENT SPECIFICATION

(11) **EP 4 073 056 B1**
(45) Date of publication and mention of the grant of the patent: **17.04.2024**
(21) Application number: 20830326.3
(22) Date of filing: 10.12.2020
(51) Int. Cl.: C07D 401/06

(54) **PROCESS FOR THE PREPARATION OF LASMIDITAN AND OF A SYNTHESIS INTERMEDIATE**
VERFAHREN ZUR HERSTELLUNG VON LASMIDITAN UND EINES SYNTHESEZWISCHENPRODUKTES
PROCÉDÉ DE PRÉPARATION DE LASMIDITAN ET D'UN INTERMÉDIAIRE DE SYNTHÈSE

(30) Priority: 13.12.2019 IT 201900023937
(43) Date of publication of application: 19.10.2022
(73) Proprietor: OLON S.p.A., 20053 Rodano (MI) (IT)
(72) Inventor: CREMONESI, Giuseppe, 20053 Rodano (MI) (IT); INVERNIZZI, Christian, 20053 Rodano (MI) (IT); SADA, Mara, 20053 Rodano (MI) (IT); FELICIANI, Lazzaro, 20053 Rodano (MI) (IT); BERTOLINI, Giorgio, 20053 Rodano (MI) (IT)
(74) Representative: Bottero, Carlo
(86) International application number: PCT/IB2020/061769
(87) International publication number: WO 2021/116979

(56) References cited:
- WO-A1-03/084949
- WO-A1-2011/123654
- MOHAMMED K. ELMKADDEM ET AL: "Efficient synthesis of aminopyridine derivatives by copper catalyzed amination reactions", CHEMICAL COMMUNICATIONS, vol. 46, no. 6, 1 December 2009 (2009-12-01), pages 925-927, XP055713355, ISSN: 1359-7345, DOI: 10.1039/B916569J

## Description

### Abstract of the Invention

The present invention refers to a process for the preparation of Lasmiditan and of a synthesis intermediate.

### Technical field

Lasmiditan, an agonist of 5-HT1F receptors present both in the central and peripheral nervous systems, has been recently approved by US Food and Drug Administration (FDA) and is marketed in the United States under the trademark of Reyvow^{®}.

Its structural Formula is the following Formula (I)

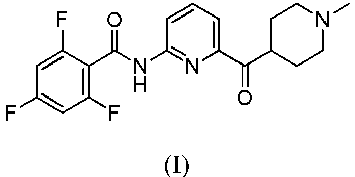

Said drug is the first of a novel class of agents for the treatment of migraine, presenting good safety and efficiency in patients with increased cardiovascular risk; the molecule reduces the release of neuropeptides and affects the pain routes without causing vasoconstriction.

Currently the drugs commonly used to treat migraine, comprising the triptans and the nonsteroidal anti-inflammatory drugs (NSAIDs) have contraindication in many patients with cardiovascular risks.

Lasmiditan and its synthetic route have been described and claimed for the first time in the Patent Application WO2003/084949 in the name of Eli Lilly. The synthetic route used in WO2003/084949, reported in the following, provides for 5 steps, as depicted in Scheme (I):

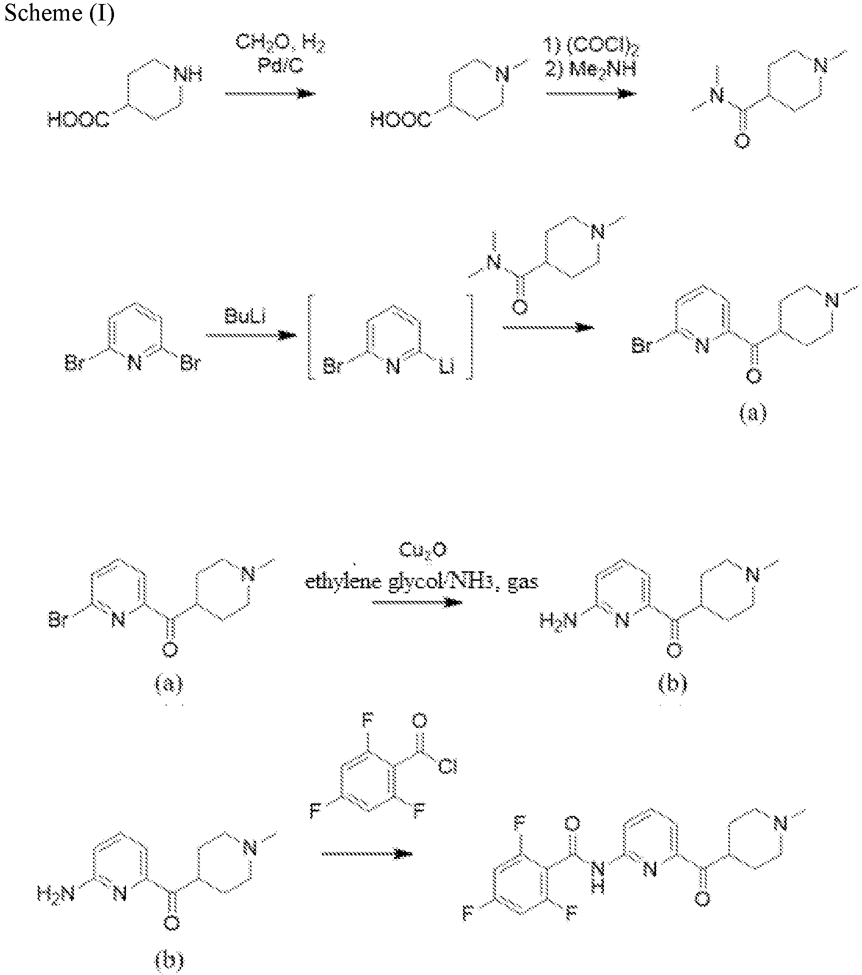

Eli Lilly has later filed a Patent Application, WO2011/123654, claiming a process boasting advancements with respect to the previous Application, in terms of yield and industrial applicability; in said second Patent Application the step of transforming the salified intermediate (a) into the intermediate (b), which are reported in Scheme (I), is carried out still in presence of an ethylene glycol solution saturated with gaseous ammonia, as already in the previous Application (WO2003/084949), but at temperatures lower than 80°C and using higher amounts of copper catalyst.

The reactions employing gaseous ammonia, in the present case ethylene glycol solutions saturated with gaseous ammonia in the reaction from intermediate (a) to intermediate (b), have difficulties related both to the preparation of said solutions and their use. As described in both the Patent Applications mentioned above, gaseous ammonia is bubbled and dissolved in ethylene glycol, said ethylene glycol being in an amount equal to 8.9 parts in weight with respect to 1 part in weight of the starting substrate (intermediate (a)). The reaction suspension comprising said starting substrate, a copper-based catalyst and the ethylene glycol solution gassed with ammonia is heated, in a closed reactor, to a pressure of 4-5 bars and kept under these conditions for not less than 10 hours. The reaction is then cooled to 15-25°C and diluted with a 16% w/w aqueous solution of sodium hydroxide and then with a 30% w/w aqueous solution of sodium chloride. The product is finally extracted three times with methyl-t-butyl ether (MTBE).

The Applicant tried to reproduce in its laboratories the above described reaction and observed that the final extraction step proved to be particularly difficult since the copper residues deriving from the catalyst, which are present in the solution, created dark sludge which significantly complicated the separation of the aqueous and organic phases, since said sludge placed themselves at the interface between the two phases. Moreover, the high water solubility of glycol and its ability to create emulsions in water made difficult the transfer of the desired product from the aqueous phase to the organic phase.

Another drawback is related to the use of gaseous ammonia, which is a toxic gas, difficult to be dosed and unhandy.

There is therefore the need of overcoming the drawbacks of the prior art to provide an alternative synthetic route for the production of a key intermediate in the preparation of Lasmiditan which is of simple processing and which does not include particularly toxic reagents.

### Objects of the invention

It is an object of the present invention to provide a process for the production of a key intermediate in the preparation of Lasmiditan.

It is another object of the invention to provide a synthetic route alternative with respect to those known for the preparation of Lasmiditan.

### Description of the invention

Subject-matter of the present invention is a process for the preparation of the compound of Formula (II) or of a salt and/or solvate thereof: 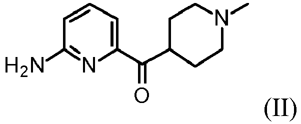 comprising reacting a compound of Formula (III) 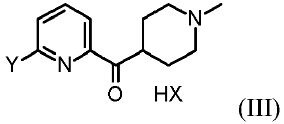
wherein X and Y, each independently, represent a halogen atom,
with an aqueous solution of ammonia, in presence of at least one bidentate ligand and at least one copper-based catalyst, and optionally converting the so-obtained compound of Formula (II) into a salt and/or solvate thereof.

According to a preferred embodiment, X and Y, each independently, represent a halogen atom selected from chlorine and bromine. X is preferably a bromine atom. Y is preferably a bromine atom. Both X and Y are more preferably a bromine atom.

Said ammonia aqueous solution is commercially available and has generally an ammonia concentration of 28-32%. Advantageously, the ammonia aqueous solution is used in a molar excess with respect to the compound of Formula (III), e.g. in a 1:20 ratio, preferably 1:25 or more with respect to the compound of Formula (III) (i.e. 20-25 moles or more of ammonia for each mole of compound of Formula (III)).

According to a preferred embodiment, said at least one bidentate ligand is selected from N-methylethanolamine, N-methylethylenediamine, N,N-dimethylethanolamine, N,N,N,N,-tetramethylpropylenediamine (TMPDA), more preferably the ligand is N-methylethanolamine.

According to a preferred embodiment, said at least one bidentate ligand is used in an amount from 0.1 to 0.2 molar equivalents with respect to the compound of Formula (III), advantageously 0.1 equivalents.

According to a particularly preferred embodiment, said at least one bidentate ligand is N-methylethanolamine used in an amount from 0.1 to 0.2 molar equivalents with respect to the compound of Formula (III), advantageously in an amount of 0.1 equivalents.

According to a preferred embodiment, said at least one copper-based catalyst is selected from CuBr, CuCl and CuzO. Preferably said at least one catalyst is used in amounts lower than 0.02 wt% with respect to the weight of the compound of Formula (III), preferably from 0.001 wt% to 0.02 wt%, e.g. from 0.005 wt% to 0.015 wt%.

According to a preferred embodiment, the process of the invention is carried out without ethylene glycol, which as set forth above, causes drawbacks in particular during the isolation step of the compound of Formula (II).

The process of the invention is carried out in the ammonia aqueous solution, which acts also as a solvent and therefore the addition of any further solvent is not required. However, if desired and necessary, it is possible to carry out the reaction also in presence of solvents, with the exclusion of ethylene glycol.

According to a preferred embodiment, the process of the invention is carried out at a temperature lower than 80°C, preferably from 55°C to 75°C, e.g. about 70°C. Optionally, at the end of the reaction the compound of Formula (II) can be isolated according to the conventional techniques, preferably in salified form obtained by reacting with organic or inorganic acids, e.g. selected from HBr, H₂SO₄, oxalic acid, p-toluenesulfonic acid. Preferably, the compound of Formula (II) is isolated after salification with an inorganic acid, advantageously hydrochloric acid to form the dihydrochloride. The compound of Formula (II) is a versatile intermediate useful in synthetic processes, particularly but without limitation useful in the preparation of Lasmiditan.

Therefore, it is a further subject-matter of the invention a process for the preparation of Lasmiditan or of a salt and/or solvate thereof, comprising reacting the compound of Formula (II), obtained by means of the process described above, with a compound of Formula (IV) 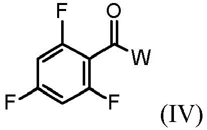 wherein W is selected from a halogen atom and an OR group, where R is selected from an alkyl group, an aryl group and a benzyl group or, alternatively, W represents a moiety of the following Formula 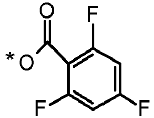 wherein the star represents the atom connecting said moiety to the carbonyl of the compound of Formula (IV), to form 2,4,6-trifluorobenzoic acid anhydride, isolating the so-obtained Lasmiditan and optionally converting it into a salt and/or solvate thereof. According to the present invention, the halogen atom is preferably a chlorine atom. According to the present invention, the alkyl group is preferably a linear or branched saturated C1-C6 alkyl group, advantageously a C1-C4 alkyl group.

According to the present invention, the aryl group is preferably a substituted or not substituted, advantageously not substituted, phenyl.

According to a preferred embodiment, the compound of Formula (IV) is 2,4,6-trifluorobenzoyl chloride.

The reaction described above can be carried out in a conventional way, according to the techniques known to the one skilled in the art.

The starting compound of Formula (III) is known in the art and can be synthesized with conventional methods.

By way of example, said compound of Formula (III) can be prepared according to the following Scheme (II): 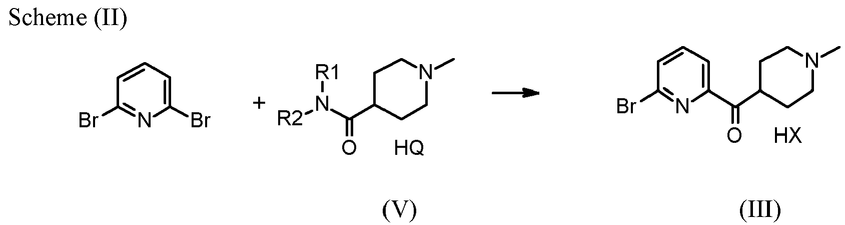 wherein R1 and R2 are, each independently, a linear or branched and saturated C1-C4 alkyl group; the compound of Formula (V) is salified with HQ, where HQ is advantageously an inorganic or organic acid, preferably an organic acid selected from oxalic acid, tartaric acid and fumaric acid, advantageously oxalic acid; the compound of Formula (III) is salified with HX, where HX is advantageously an inorganic acid, preferably hydrobromic acid.

The process of the invention allows to obtain the compound of Formula (II) and Lasmiditan with good yields and high purity.

As it is clear, the use of an ammonia aqueous solution with respect to gaseous ammonia in ethylene glycol, as described in the known art, makes the process more easily applicable to an industrial production scale.

Still with reference to known processes, with the process of the invention it has been possible to obtain improvements, in terms of overall yield and process costs, in addition to an increase of the purity and yield of the desired product.

Moreover the use of aqueous ammonia without ethylene glycol allowed to further reduce the amounts of catalyst used with respect to known processes and to work at temperatures lower than 80°C, thus achieving a reduction of the costs of the process also in this way. The invention will be now described in detail through the following examples, by illustrative way only and without limitation.

### Experimental Section

### Example 1 Synthesis of N,N-diethyl-1-methylpiperidin-4-carboxamide oxalate (Formula (V), R1 and R2 = ethyl; HQ = oxalic acid)

1-methylpiperidin-4-carboxylic acid hydrochloride (84.0 g, 0.468 moles) is suspended in a glass reactor with methylene chloride (695 mL) and N,N-dimethylformamide (72 mL). To the suspension heated to 30-35°C thionyl chloride (46.1 mL, 0.635 moles) is added in about 20-30 min. The mixture is brought to the reflux temperature (40-45°C) for 2 hours. The solvent is distilled at ambient pressure. The suspension is diluted with toluene (690 mL) and the suspension is brought to 60 ±5°C. Afterwards, diethylamine is added (210 mL, 2 moles). The reaction is kept under stirring at 60 ±5°C for 2 hrs and then it is left under stirring at room temperature for 16-18 hrs. The mixture is diluted with water (140 mL) and is transferred into a rector containing a mixture of sodium chloride (10.0 g) and sodium hydroxide (30 wt% solution, 180 mL, 1.8 moles). The aqueous phase is separated and extracted with toluene (414 mL). The organic phases are combined, filtered and the solvent evaporated under reduced pressure. On the residue, taken up with 2-propanol (564 mL) and brought to 50 ± 5°C, a solution of oxalic acid (42 g, 0.466 moles) in 2-propanol (253 mL) is dropped. The resulting suspension is maintained at 50 ± 5°C for 1 hour and then cooled to 15-20°C and kept for 1-2 hours. The solid is recovered by filtration and washed with 2-propanol. The solid is dried at 65°C for 16-18 hours, obtaining 125.8 g of a clear brown solid. Yield 93.0%.

¹H-NMR (300 MHz, DMSO): 9.78 (2H, *br.s);* 3.75-3.19 (6H, *m);* 2.92 (2H, *m);* 2.73 (1H, *m*)*;* 2.66 (3H, *s*)*;* 1.79 (4H, *m);* 1.11 (3H, *t, J*= 7.0 Hz); 0.98 (3H, *t, J*= 7.0 Hz) ppm.

### Example 2

### Synthesis of (6-bromopyridin-2-yl)(1-methylpiperidin-4-yl)methanone hydrobromide (Formula (III); HX = hydrobromic acid)

*N,N-diethyl-1-methylpiperidin-4-carboxamide* oxalate (102.0 g, 0.354 moles) is charged into a glass reactor containing a solution of potassium carbonate (146.0 g, 1.057 moles) in water (430.0 mL). After 5 minutes stirring, methylene chloride (323 mL) is added and the stirring is maintained at 20-25°C for 20 min. The phases are separated and the aqueous phase is treated with methylene chloride (323 mL). The combined organic phases are evaporated under vacuum. The residue dissolved in 2-Me-THF (224 mL) is maintained at 20-25°C and used in the next step. 2,6-Dibromopyridine (121.38 g, 0.512 moles) is dissolved in 2-Me-THF (534 mL) in a glass reactor and cooled to -65 ± 5°C. A solution of n-hexyllithium in hexane (2.47 M, 200 mL, 0.494 moles) is added in about 1 hour by maintaining the temperature at -65 ± 5°C. The solution is maintained at -65 ± 5°C for 1 hour. The solution of *N,N*-diethyl-1-methylpiperidin-4-carboxamide oxalate free base is added to the solution of lithiated 2,6-dibromopyridine in about 1 hour by maintaining the temperature at -65 ± 5°C. The reaction is maintained at -65 ± 5°C for 4 hours. The reaction is left to recover at about -40 ± 5°C and the reaction is quenched by adding ammonium chloride (10 wt% solution, 427 mL). The reaction is left under stirring at 20-25°C for 2-4 hours. The reaction is brought to pH 7.6-7.7 by adding hydrochloric acid (37 wt% solution). The phases are separated and the aqueous phase is extracted with THF (3 x 225 mL). The combined organic phases are charged into a glass reactor with water (400.0 mL) and heptane (20 mL). The pH is brought to 1.0-1.5 by adding hydrochloric acid (37 wt% solution). The phases are separated and the organic phase is extracted with hydrochloric acid (10 wt% solution, 2 x 400 mL). The aqueous phases are combined and basified to pH 10.5-11.5 by adding sodium hydroxide (30 wt% solution). The aqueous phase is extracted with isobutanol (3 x 200 mL). The organic phases are combined and added with ammonium bromide (52 g, 0.532 moles). The solvent is distilled under ambient pressure up to 105-107°C in a boiler, followed by slow cooling. The resulting dispersion is kept at 20-25°C for 1-2 hours, and then cooled to 0-5°C and kept for 1-2 hours. The solid is recovered by filtration and the filtrate is washed with isobutanol. The solid is dried at 65°C for 16-18 hours under vacuum, obtaining 113.9 g of a brown/green solid. Yield 88.4%.

¹H-NMR (300 MHz, DMSO): 9.48 (1H, *br.s*)*;* 7.97 (3H, *m);* 3.90 (1H, *m*)*;* 3.49 (2H, *m*); 3.16 (2H, *m*); 2.76 (3H, *d, J*= 4.35 Hz); 2.05 (2H, *m*); 2.78 (2H, *m*) ppm.

### Example 3

### Synthesis of (6-aminopyridin-2-yl)(1-methylpiperidin-4-yl)methanone (Formula (II)) dihydrochloride

(6-Bromopyridin-2-yl)(1-methylpiperidin-4-yl)methanone HBr (53.0 g, 0.146 moles) is suspended in a steel autoclave with ammonium hydroxide (28 wt% solution, 245 mL, 3.62 moles), N methylethanolamine (1.13 mL, 0.014 moles) and Cu₂O (0.015 wt%, 0.795 g, 5.54 mmoles). The autoclave is heated to 70-75°C up to the internal pressure of 3-4 bars. The reaction is kept under stirring at 70-75°C for 16-18 hours. The reaction is cooled to 20-25°C and diluted with water (80 mL). Sodium chloride (50 g), Na₂S (60 %, 4.5 g, 0.035 moles), activated carbon (5.3 g), sodium hydroxide (30 wt% solution, 15 mL) and ethyl acetate (220 mL) are added. It is left under stirring for 1 hour and the suspension is filtered on cellulose. The filter is washed with ethyl acetate (110 mL). The phases are separated and the aqueous phase is extracted with ethyl acetate (2 x 220 mL). The organic phases are combined and the solvent is evaporated under vacuum. The residue is taken up with 2-propanol (225 mL) and added with hydrochloric acid (37 wt% solution, 37 mL, 0.43 moles) by keeping the temperature lower than 30°C. The suspension is distilled under vacuum until residue. The raw is crystallized from hydroalcoholic mixture. 28 g of a solid are obtained. Yield 66%.

¹H-NMR (300 MHz, DMSO): 11.05 (1H, *br.s*)*;* 9.74-7.25 (2H, *br.s*)*;* 8.01 (1H, *m*)*;* 7.69 (1H, *d, J*= 6.98 Hz); 7.30 (1H, *d, J*= 8.68 Hz); 3.83-3.21 (5H, *m*)*;* 3.04 (2H, *m*); 2.70 (3H, *d, J*= 4.63 Hz); 1.95 (2H, *m*) ppm.

### Example 4

### Synthesis of (6-aminopyridin-2-yl)(1-methylpiperidin-4-yl)methanone (Formula (II)) dihydrochloride

(6-Bromopyridin-2-yl)(1-methylpiperidin-4-yl)methanone HBr (53.0 g, 0.146 moles) is suspended in a steel autoclave with ammonium hydroxide (28 wt% solution, 245 mL, 3.62 moles), *N*-methylethanolamine (1.13 mL, 0.014 moles) and CuzO (0.005 wt%, 0.265 g, 1.85 mmoles). The autoclave is heated to 70-75°C up to the internal pressure of 3-4 bars. The reaction is kept under stirring at 70-75°C for 16-18 hours. The reaction is cooled to 20-25°C and diluted with water (80 mL). Sodium chloride (50 g), Na₂S (60 %, 4.5 g, 0.035 moles), activated carbon (5.3 g), sodium hydroxide (30 wt% solution, 15 mL) and ethyl acetate (220 mL) are added. It is left under stirring for 1 hour and the suspension is filtered on cellulose. The filter is washed with ethyl acetate (110 mL). The phases are separated and the aqueous phase is extracted with ethyl acetate (2 x 220 mL). The organic phases are combined and the solvent is evaporated under vacuum. The residue is taken up with 2-propanol (225 mL) and added with hydrochloric acid (37 wt% solution, 37 mL, 0.43 moles) by keeping the temperature lower than 30°C. The suspension is distilled under vacuum until residue. The raw is crystallized from hydroalcoholic mixture. 22.8 g of a solid are obtained. Yield 53.6%.

### Example 5

### Synthesis of 2,4,6-trifluoro-N-[6-(1-methyl-piperidin-4-ylcarbonyl)-pyridin-2-yl]-benzamide (Formula (I)) hydrochloride

(6-Aminopyridin-2-yl)(1-methylpiperidin-4-yl)methanone dihydrochloride (27.7 g, 0.095 moles) is suspended in sodium hydroxide (30 wt% solution, 21 mL) and water (21 mL) at 20-25°C. The product is extracted with methylene chloride (2 x 140 mL). After evaporation, the residue is dissolved in THF (93.4 mL) and the solution is cooled to 0-5°C. Triethylamine is charged (33.3 mL, 0.24 moles). 2,4,6-trifluorobenzoyl chloride (16.8 mL, 0.128 moles) is dropped in about 1 hour by keeping the temperature at 0-5°C. Stirring is maintained at 0-5°C for 2 hours and then at 20-25°C for 16-18 hours. The reaction is cooled to 0-10°C and water (125 mL) and sodium hydroxide (30 wt% solution, 62.5 mL) are charged by keeping under stirring for 30 minutes. The phases are separated and the aqueous solution is extracted with methylene chloride (3 x 145 mL). The combined organic phases are evaporated under vacuum until residue. The residue is taken up in 2-propanol (186 mL). Hydrochloric acid (3.8 g, 0.104 moles) dissolved in 2-propanol (31.7 mL) is charged. The suspension is brought to reflux and the reflux is maintained for about 30 min. The suspension is cooled to 20-25°C and the suspension in kept under stirring for 1-2 hours. The product is recovered by filtration. The solid is washed with 2-propanol and the solid is dried at 65°C for 16-18 hours, obtaining 32.7 g of a white solid. Yield 83.4%.

¹H-NMR (300 MHz, DMSO): 11.52 (1H, *s*)*;* 10.87 (1H, *br.s*); 8.34 (1H, *br.d, J*= 7.5 Hz); 8.07 (1H, *aromatic t, J*= 7.9 Hz); 7.75 (1H, *d, J*= 7.5 Hz); 7.39 (2H, *m*); 3.74 (1H, *br.m*); 3.37 (2H, *br.m*); 2.97 (2H, *br.m*); 2.70 (3H, *s*); 2.03 (4H, *br.m*) ppm.

## Claims

1. A process for the preparation of the compound of Formula (II) or of a salt and/or solvate thereof: 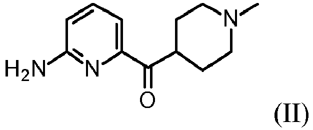 comprising reacting a compound of Formula (III) 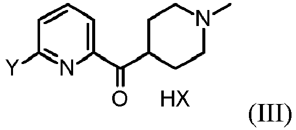 wherein X and Y, each independently, represent a halogen atom, with an aqueous solution of ammonia in the absence of ethylene glycol, in the presence of at least one bidentate ligand and of at least one copper-based catalyst, and optionally converting the so-obtained compound of Formula (II) into a salt and/or solvate thereof.

2. The process according to claim 1, **characterized in that** X and Y, each independently, represent a halogen atom selected from chlorine and bromine.

3. The process according to claim 2, **characterized in that** X and Y each represents a bromine atom.

4. The process according to any one of claims 1 to 3, **characterized in that** the ammonia in said aqueous solution of ammonia is used in molar excess with respect to the compound of Formula (III).

5. The process according to any one of claims 1 to 4, **characterized in that** said at least one bidentate ligand is selected from N-methylethanolamine, N-methylethylenediamine, N,N-dimethylethanolamine and N,N,N,N,-tetramethylpropylenediamine (TMPDA), preferably N-methylethanolamine.

6. The process according to any one of claims 1 to 5, **characterized in that** said at least one bidentate ligand is used in an amount from 0.1 to 0.2 molar equivalents with respect to the compound of Formula (III), preferably 0.1 equivalents.

7. The process according to any one of claims 1 to 6, **characterized in that** said at least one copper-based catalyst is selected from CuBr, CuCl and Cu₂O preferably Cu₂O.

8. The process according to any one of claims 1 to 7, **characterized in that** said at least one catalyst is used in an amount from 0.001 wt% to 0.02 wt% with respect to the weight of the compound of Formula (III).

9. A process for the preparation of Lasmiditan of Formula (I) 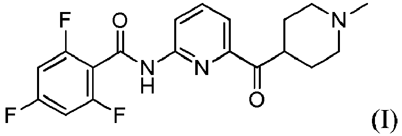 or of a salt or solvate thereof, comprising the process of claims 1-8 and further comprising reacting the compound of Formula (II) a compound of Formula (IV) 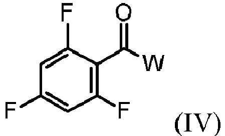 wherein W is selected from a halogen atom and an OR group, where R is selected from an alkyl group, an aryl group and a benzyl group or, alternatively, W represents a moiety of the following Formula 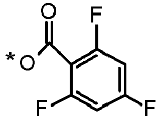 wherein the star represents the atom connecting said moiety to the carbonyl of the compound of Formula (IV), to form 2,4,6-trifluorobenzoic acid anhydride, isolating the so- obtained Lasmiditan and optionally converting it into a salt and/or solvate thereof.

## Patentansprüche

1. Verfahren zur Herstellung der Verbindung der Formel (II) oder eines Salzes und/oder Solvats davon: 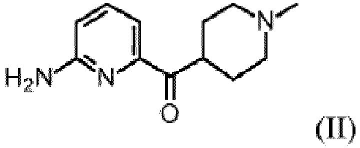 umfassend die Umsetzung einer Verbindung der Formel (III) 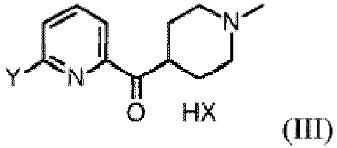 worin X und Y jeweils unabhängig voneinander ein Halogenatom darstellen, mit einer wässrigen Lösung von Ammoniak in Abwesenheit von Ethylenglykol, in Gegenwart von mindestens einem zweizähnigen Liganden und mindestens einem Katalysator auf Kupferbasis und gegebenenfalls Umwandlung der so erhaltenen Verbindung der Formel (II) in ein Salz und/oder Solvat davon.

2. Verfahren nach Anspruch 1, **dadurch gekennzeichnet, dass** X und Y jeweils unabhängig voneinander ein Halogenatom, ausgewählt aus Chlor und Brom, darstellen.

3. Verfahren nach Anspruch 2, **dadurch gekennzeichnet, dass** X und Y jeweils ein Bromatom darstellen.

4. Verfahren nach einem der Ansprüche 1 bis 3, **dadurch gekennzeichnet, dass** das Ammoniak in der wässrigen Ammoniaklösung im molaren Überschuss in Bezug auf die Verbindung der Formel (III) verwendet wird.

5. Verfahren nach einem der Ansprüche 1 bis 4, **dadurch gekennzeichnet, dass** der mindestens eine zweizähnige Ligand ausgewählt ist aus N-Methylethanolamin, N-Methylethylendiamin, N,N-Dimethylethanolamin und N,N,N,N,-Tetramethylpropylendiamin (TMPDA), vorzugsweise N-Methylethanolamin.

6. Verfahren nach einem der Ansprüche 1 bis 5, **dadurch gekennzeichnet, dass** der mindestens eine zweizähnige Ligand in einer Menge von 0,1 bis 0,2 Moläquivalenten, bezogen auf die Verbindung der Formel (III), verwendet wird, vorzugsweise 0,1 Äquivalente.

7. Verfahren nach einem der Ansprüche 1 bis 6, **dadurch gekennzeichnet, dass** der mindestens eine Katalysator auf Kupferbasis ausgewählt ist aus CuBr, CuCI und CuzO, vorzugsweise CuzO.

8. Verfahren nach einem der Ansprüche 1 bis 7, **dadurch gekennzeichnet, dass** der mindestens eine Katalysator in einer Menge von 0,001 Gew.-% bis 0,02 Gew.-%, bezogen auf das Gewicht der Verbindung der Formel (III), verwendet wird.

9. Verfahren zur Herstellung von Lasmiditan von Formel (I) 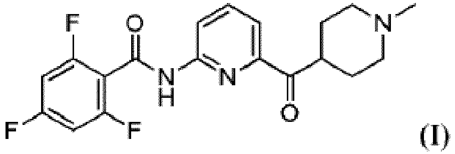 oder eines Salzes oder Solvats davon, umfassend das Verfahren nach den Ansprüchen 1 bis 8 und ferner umfassend die Umsetzung der Verbindung der Formel (II) mit einer Verbindung der Formel (IV), 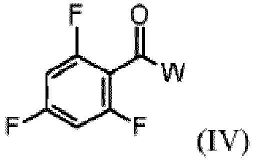 worin W aus einem Halogenatom und einer OR-Gruppe ausgewählt ist, wobei R aus einer Alkylgruppe, einer Arylgruppe und einer Benzylgruppe ausgewählt ist oder alternativ W eine Einheit der folgenden Formel darstellt 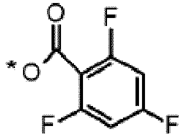 worin der Stern das Atom darstellt, das diese Einheit mit dem Carbonyl der Verbindung der Formel (IV) verbindet, um 2,4,6-Trifluorbenzoesäureanhydrid zu bilden, das so erhaltene Lasmiditan zu isolieren und es gegebenenfalls in ein Salz und/oder Solvat davon umzuwandeln.

## Revendications

1. Procédé pour la préparation du composé de Formule (II) ou d'un sel et/ou solvate de celui-ci: 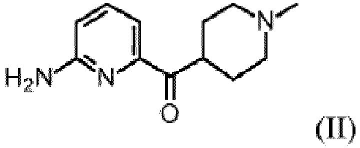 comprenant la mise en réaction d'un composé de Formule (III) 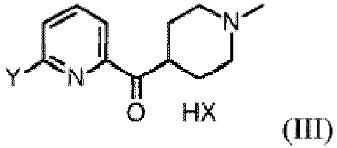 dans lequel X et Y, chacun indépendamment, représentent un atome d'halogène, avec une solution aqueuse d'ammoniac en l'absence d'éthylène glycol, en présence d'au moins un ligand bidentate et d'au moins un catalyseur à base de cuivre, et facultativement la conversion du composé de Formule (II) ainsi obtenu en un sel et/ou solvate de celui-ci.

2. Procédé selon la revendication 1, **caractérisé en ce que** X et Y, chacun indépendamment, représentent un atome d'halogène sélectionné parmi le chlore et le brome.

3. Procédé selon la revendication 2, **caractérisé en ce que** X et Y représentent chacun un atome de brome.

4. Procédé selon l'une quelconque des revendications 1 à 3, **caractérisé en ce que** l'ammoniac dans ladite solution aqueuse d'ammoniac est utilisé en excès molaire par rapport au composé de Formule (III).

5. Procédé selon l'une quelconque des revendications 1 à 4, **caractérisé en ce que** ledit au moins un ligand bidentate est sélectionné parmi la N-méthyléthanolamine, la N-méthyléthylènediamine, la N,N-diméthyléthanolamine et la N,N,N,N,-tétraméthylpropylènediamine (TMPDA), de préférence la N-méthyléthanolamine.

6. Procédé selon l'une quelconque des revendications 1 à 5, **caractérisé en ce que** ledit au moins un ligand bidentate est utilisé dans une quantité de 0,1 à 0,2 équivalents molaires par rapport au composé de Formule (III), de préférence 0,1 équivalents.

7. Procédé selon l'une quelconque des revendications 1 à 6, **caractérisé en ce que** ledit au moins un catalyseur à base de cuivre est sélectionné parmi CuBr, CuCI et CuzO, de préférence CuzO.

8. Procédé selon l'une quelconque des revendications 1 à 7, **caractérisé en ce que** ledit au moins un catalyseur est utilisé dans une quantité de 0,001 % en poids à 0,02 % en poids par rapport au poids du composé de Formule (III).

9. Procédé pour la préparation de Lasmiditan de Formule (I) 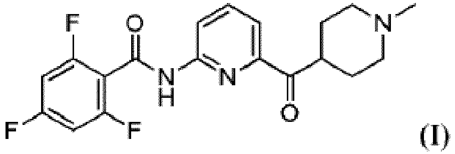 ou d'un sel ou solvate de celui-ci, comprenant le procédé selon les revendications 1 à 8 et comprenant en outre la mise en réaction du composé de Formule (II) avec un composé de Formule (IV) 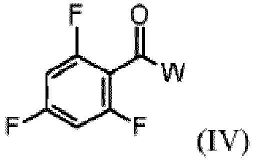 dans lequel W est sélectionné parmi un atome d'halogène et un groupe OR, où R est sélectionné parmi un groupe alkyle, un groupe aryle et un groupe benzyle ou, en variante, W représente une fraction de la Formule suivante 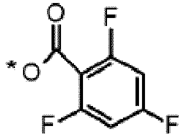 dans lequel l'étoile représente l'atome reliant ladite fraction au carbonyle du composé de Formule (IV), pour former de l'anhydride d'acide 2,4,6-trifluorobenzoïque, l'isolement du Lasmiditan ainsi obtenu et facultativement sa conversion en un sel et/ou solvate de celui-ci.
